# EUROPEAN PATENT APPLICATION

(11) **EP 1 774 974 A1**
(43) Date of publication of application: **18.04.2007**
(21) Application number: 05090285.7
(22) Date of filing: 13.10.2005
(51) Int. Cl.: A61K 36/38, A61K 31/12, A61P 3/00

(54) **Use of hyperforin or St. John's Wort in the treatment and prevention of metabolic syndrome**

(71) Applicant: Roots, Ivar, 10117 Berlin (DE)
(72) Inventor: Roots, Ivar, Prof. Dr., 10117 Berlin (DE); Krusekopf, Solveigh, Dr., 13158 Berlin (DE)
(74) Representative: Ziebig, Marlene

(57) **Abstract**

The invention relates to the use of hyperforin, its derivatives or a hyperforin-containing extract of Hypericum perforatum L. (St. John's Wort) for the manufacture of a pharamaceutical composition for the treatment or prevention of Metabolic Syndrome and to a method of treating or preventing Metabolic Syndrome by administering to a patient in need of such treatment an effective amount of hyperforin, its derivatives or a hyperforin-containing extract of Hypericum perforatum L (St. John's Wort).

## Description

The invention relates to the use of hyperforin, its derivatives or a hyperforin-containing extract of Hypericum perforatum L. (St. John's Wort) for the manufacture of a pharamaceutical composition for the treatment or prevention of Metabolic Syndrome and to a method of treating or preventing Metabolic Syndrome by administering to a patient in need of such treatment an effective amount of hyperforin, its derivatives or a hyperforin-containing extract of Hypericum perforatum L (St. John's Wort). Metabolic Syndrome is a medical condition characterized by a cluster of metabolic disorders such as central obesity, elevated insulin, elevated triglycerides, low HDL, high blood pressure and glucose intolerance.

It is well known in the literature that the herbal remedy St. John's Wort (Hypericum perforatum L.) is useful for the treatment of depression and psychovegetative disorders. In addition, it exhibits antimicrobial activity and inhibits the growth of tumor cells by induction of apoptosis.

St. John's Wort contains a number of classes of structurally different substances. Hyperforin, a phloroglucin derivative, is one of the main components of the lipophilic fraction of Hypericum perforatum flowering tops; said fraction also contains adhyperforin, a hyperforin higher homologue.

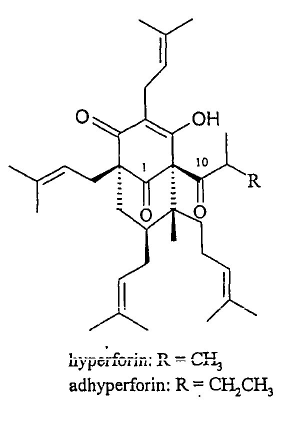

Hyperforin is known to be useful in the treatment of tumor diseases (DE 199 13 333 A1), anaphylactic shock and osteoporosis (WO 03/053456 A1) or dementia diseases such as for example Alzheimer's disease (US 6,322,824 B1).

Surprisingly, it has now been found that hyperforin, adhyperforin or their derivatives, such as for example salts or esters, as well as hyperforin-containing extracts of Hypericum perforatum L. can be used for the treatment and prevention of Metabolic Syndrome which is also known as Insulin Resistance Syndrome or Dysmetabolic Syndrome.

Gene expression investigations with hyperforin-containing extracts of St. John's Wort and with hyperforin itself have shown an upregulation of glycolytic genes and glucose transporters indicating an increased glucose metabolism resulting in a decrease of glucose level and a downregulation of genes involved in cholesterol biosynthesis, resulting in a decrease of cholesterol level. Such effects have not been described previously neither for hyperforin nor for hyperforin-containing extracts and were also not expected.

According to the invention each commercially available St. John's Wort dry extract such as for instance Esbericum^{®}-capsules (Schaper und Brümmer, Salzgitter, Deutschland) can be used for the treatment of Metabolic Syndrome, provided the dry extracts contain at least 2 % by weight of hyperforin. DE-A-196 19 512 and DE-A-197 14 450 describe extracts with a stable amount of hyperforin which is usually an instable component of St. John's Wort. These extracts as well as the extracts of DE-A-199 03 570 are useful according to the invention. DE-A-199 03 570 discloses hyperforin formulations wherein the hyperforin is stabilized by a complexing agent. Dry extracts of hypericum which have a higher content of hyperforin than the vegetable starting material are disclosed in EP-A-0 599 307. These extracts can also be used for the treatment according to the invention.

The instability of hyperforin or adhyperforin makes the preparation of hyperforin pharmaceutical formulations quite difficult. Therefore, more stable derivatives such as for instance the salts with inorganic cations or ammonium salts described in WO 99/41 220 are preferably used.

The described hyperforin-containing extracts, hyperforin or its derivatives are formulated to pharmaceutical compositions in a known manner with common carriers, diluents and/or additives. These pharmaceutically acceptable excipients comprise salts, buffers, fillers, chelating agents, antioxidants, solvents, bonding agents, lubricants, tablet coatings, flavor additives, flavors, preservatives and suspending agents. In a preferred embodiment of the invention the formulation is administered orally as a tablet, film tablet, capsule, pill, powder, solution, dispersion or suspension.

The pharmaceutical compositions contain at least 2 % by weight of hyperforin, preferably 2 to 5 % by weight. It will be understood that the specific dose level, frequency and period of administration of the hyperforin-containing extract or the hyperforin to any particular human subject will depend upon the age, body weight and severity of the Metabolic Syndrome. In an embodiment of the present invention a hyperforin extract with 5 % hyperforin is administered in an amount of 300-400 mg three times a day.

The entire disclosure of all applications and patents cited above or below is hereby incorporated by reference.

The following investigations illustrate the present invention in further detail.

### Examples

Effects of St. John's wort and hyperforin on gene expression were analysed in HepG2 cells by Affymetrix microarray hybridisation and real time RT-PCR. Expression of genes mediating cholesterol biosynthesis was decreased by both compounds, while facilitated glucose transporters and glycolysis genes were induced, indicating increased glucose metabolism. Thus, St. John's wort and hyperforin concordantly affected expression of genes involved in energy metabolism and cell proliferation.

### Methods

### Cell culture and drug treatment

HepG2 cells (human hepatocellular carcinoma, German Collection of Microorganisms and Cell Cultures, DSMZ, Braunschweig, Germany) were grown in Dulbecco's Modified Eagle Medium containing 1 g/l D-glucose and 5% fetal bovine serum with 5% CO₂ at 37°C. Cells were seeded in 6-well dishes and grown to confluence.
Content of 1 Esbericum^{™} capsule (total hypericin 250 mg, Schaper & Bruemmer, Salzgitter, Germany) was extracted with 1 ml ethanol. Cells were treated with either this extract (final concentration 0.1% (v/v)), or 1 µM hyperforin (Calbiochem, Schwalbach, Germany), or 0.1 % ethanol (controls) for 24 h. Three independent experiments were performed.

### Harvest of cells and RNA preparation

Cells were washed twice with phosphate buffered saline, harvested in lysis buffer (Qiagen, Hilden, Germany) with a rubber-policeman and homogenised by centrifugation through a Qiashredder (Qiagen, Hilden, Germany). Preparation of total RNA was performed using a Qiagen RNeasy Mini Kit according to the manufacturer's instructions. RNA concentration was determined photometrically (Uvicon photometer, Kontron). RNA was either used immediately for cDNA synthesis or stored at -80°C.

### Microarray analysis

Expression analysis was performed with Affymetrix HG U95Av2 microarray (Affymetrix, Santa Clara, CA). Synthesis of biotin-labelled cRNA and hybridisation were carried out following the Affymetrix protocol. Results were exported to Excei for analysis.

### Reverse transcription, Real-time PCR

5 µg RNA was reverse transcribed in a total volume of 20 µl containing 200 U SuperScript^{™} II RT and 1x reaction buffer (Invitrogen, Karlsruhe, Germany), 500 ng pd(T)₁₂₋₁₈ (Amersham Pharmacia Biotech, Freiburg, Germany) and 125 pmol/µl of each dNTP at 42°C for 1 h.
Real-time PCR was performed with QuantiTect^{™} SYBR^{®} Green PCR Kit (Qiagen, Hilden, Germany) according to the manufacturer's instructions. The reaction was carried out in a LightCycler (Roche, Mannheim, Germany), version 5.32, and analysed using the Second Derivative Maximum method.

### Results

### Comparison of microarray- and RT-PCR results

HepG2 cells were treated with either extract of Hypericum perforatum (St. John's wort, SJW), hyperforin, or ethanol (control) for 24 h. The hyperforin concentration given to the cells was 1 µM; this is equivalent to 536.8 ng/ml. Hyperforin content of the SJW-preparation was 560 µg, resulting in a final concentration of 560 ng/ml hyperforin in the culture medium. Thus, equal hyperforin concentrations were administered by both treatments.
Affymetrix microarray hybridisation was used to measure the effects of both treatments on mRNA abundance. 182 (SJW Fold) resp. 201 (hyperforin Fold) sequences were identified as differentially regulated (p<0.05). 101 (SJW Fold) and 120 (hyperforin Fold) of these were upregulated. 91 sequences met the selection criteria in both treatments, 64 upregulated and 27 downregulated in both cases. There was no sequence contrariwise regulated by SJW and hyperforin. Hence, SJW and hyperforin affected gene expression similarly.
The array-derived expression fold change values of 25 genes were verified by RT-PCR. (Table 1). The correlation coefficient was 0.991 for SJW and 0.967 for hyperforin, confirming a close match of the outcome of both methods.

### Cholesterol biosynthesis

We observed a downregulation of mevalonate pyrophosphate decarboxylase (MVD) and farnesyl diphosphate synthase (FDPS) (Table 1). Transcripts of several enzymes located at a later position of the cholesterol biosynthesis pathway (CYP51A1, DHCR24, DHCR7, EBP, FDFT1, NSDHL, LSS, SC5DL, SQLE) were marginally decreased, but did not meet the selection criteria. The mRNA coding for TM7SF2 (transmembrane 7 superfamily member 2), a protein localised in the ER (endoplasmic reticulum) membrane and homologue to 7-dehydrocholesterol reductase, was also downregulated.

### Glycolysis and glucose transport

Several mRNAs coding for glycolytic enzymes were upregulated by SJW and hyperforin (Table 1). The mitochondrial gluconeogenetic phosphoenolpyrovate carboxykinase 2 (PCK2, PEPCK2) was downregulated. The glucose transporters SLC2A3 (GLUT3) and SLC2A1 (GLUT1) were upregulated by both treatments, while the mitochondrial uncoupling protein 2 (UCP2) was downregulated. These data indicate an increase in glucose metabolism.

**Table 1. Fold change values of transcripts after treatment of cells with St. John's wort or hyperforin**

| **Gene Symbol** | **St. John's wort** | | | | **Hyperforin** | | | | **RefSeq** | **Gene Title** |
|---|---|---|---|---|---|---|---|---|---|---|
| | **Array** | | **RT-PCR** | | **Array** | | **RT-PCR** | | | |
| | **Fold** | CV | **Fold** | CV | **Fold** | CV | **Fold** | CV | | |
| **Cholesterol biosynthesis** | | | | | | | | | | |
| FDPS | **0.53** | 28.19 | **0.51** | 18.60 | **0.53** | 14.85 | **0.49** | 17.03 | NM_002004 | farnesyl diphosphate synth. (geranyltranstransferase) |
| MVD | **0.50** | 59.98 | **0.56** | 25.57 | **0.26** | 189.61 | **0.56** | 31.87 | NM_002461 | mevalonate (diphospho) decarboxylase |
| TM7SF2 | **0.57** | 14.16 | | | **0.52** | 3.82 | | | NM_003273 | transmembrane 7 superfamily member 2 |

| **Glycolysis and glucose transport** | | | | | | | | | | |
|---|---|---|---|---|---|---|---|---|---|---|
| ALDOA | **1.40** | 14.53 | **1.37** | 4.91 | **1.41** | 6.71 | **1.42** | 6.40 | NM_000034 | aldolase A, fructose-bisphosphate |
| ENO2 | **2.19** | 21.88 | | | **2.75** | 35.18 | | | NM_001975 | enolase 2, (gamma, neuronal) |
| LDHA | **1.24** | 7.04 | | | **1.36** | 18.22 | | | NM_005566 | lactate dehydrogenase A |
| LDHB | **1.39** | 21.88 | | | **1.42** | 17.98 | | | NM_002300 | lactate dehydrogenase B |
| PCK2 | **0.71** | 23.74 | **0.70** | 14.48 | **0.43** | 70.17 | **0.49** | 54.38 | NM_004563 | phosphoenolpyruvate carboxykinase 2 (mitochondrial) |
| PFKFB3 | **1.84** | 18.55 | | | **1.83** | 70.94 | | | NM_004566 | 6-phosphofructo-2-kinase/fructose-2,6-biphosphatase 3 |
| PGK1 | **1.75** | 6.51 | | | **1.50** | 35.53 | | | NM_000291 | phosphoglycerate kinase 1 |
| PKM2 | **1.41** | 13.06 | | | **1.40** | 2.02 | | | NM_002654 | pyruvate kinase, muscle |
| SLC25A8 / UCP2 | **0.65** | 16.98 | | | **0.54** | 25.04 | | | NM_003355 | uncoupling protein 2 (mitochondrial, proton carrier) |
| SLC2A1 | **1.62** | 18.10 | **1.62** | 13.21 | **1.74** | 8.75 | **1.90** | 7.87 | NM_006516 | solute carrier fam. 2 (facilitated glucose transp.), m. 1 |
| SLC2A3 | **3.05** | 11.98 | **2.53** | 28.69 | **3.57** | 3.50 | **2.84** | 19.56 | NM_006931 | solute carrier fam. 2 (facilitated glucose transp.), m. 3 |
| IGFBP3 | **4.35** | 44.68 | **4.47** | 7.68 | **8.79** | 58.71 | **8.29** | 29.27 | NM_000598 | insulin-like growth factor binding protein 3 |

| | | | | | | | | | | |
|---|---|---|---|---|---|---|---|---|---|---|
| Fold, geometric mean of fold change of treatment vs. control of three experiments; CV, coefficient of variation. | | | | | | | | | | |

From the foregoing description, one skilled in the art can easily ascertain the essential characteristics of this invention and, without departing from the spirit and scope thereof, can make various changes and modifications of the invention to adapt it to various usages and conditions.

## Claims

1. Use of hyperforin, its derivatives or a hyperforin-containing extract of Hypericum perforatum L. (St. John's Wort) for the manufacture of a pharmaceutical composition for the treatment or prevention of Metabolic Syndrome.

2. The use according to claim 1 wherein the pharmaceutical composition contains at least 2 % by weight of hyperforin.

3. A method of treating or preventing Metabolic Syndrome by administering to a patient in need of such treatment an effective amount of hyperforin, its derivatives or a hyperforin-containing extract of Hypericum perforatum L. (St. John's Wort).

4. The method according to claim 3 wherein the extract contains at least 2 % by weight of hyperforin.
